# EUROPEAN PATENT SPECIFICATION

(11) **EP 1 580 218 B1**
(45) Date of publication and mention of the grant of the patent: **09.05.2007**
(21) Application number: 05005851.0
(22) Date of filing: 17.03.2005
(51) Int. Cl.: C08J 3/03, A61K 8/06, A61K 8/70, A61Q 17/00, A61K 9/113, C08L 71/02

(54) **Complex emulsions of perfluoropolyethers**
Komplexe Emulsionen von Perfluoropolyethern
Emulsions complexes de perfluoropolyéthers

(30) Priority: 25.03.2004 IT MI20040586
(43) Date of publication of application: 28.09.2005
(73) Proprietor: Solvay Solexis S.p.A., 20121 Milano (IT)
(72) Inventor: Pantini, Giovanni, 20100 Milano (IT)
(74) Representative: Sama, Daniele

(56) References cited:
- US-A- 4 803 067
- US-B1- 6 174 518
- PANTINI, G.; INGOGLIA, F.; BRUNETTA, F.; DE ASCENTIIS, A.; BERNARDI, F.: "Protective and sunscreen emulsions containing a perfluoropolyether amide" SOFW JOURNAL, vol. 127, no. 5, May 2001 (2001-05), pages 13-17, XP001206575
- "16TH IFSCC CONGRESS" SOAP PERFUMERY AND COSMETICS, UNITED TRADE PRESS LTD. LONDON, GB, vol. 64, no. 6, 1 June 1991 (1991-06-01), page 58, XP000227208 ISSN: 0037-749X

## Description

The present invention relates to compositions in the form of emulsions having water as external phase the compositions being oleorepellent and with an improved water-repellence.

More specifically the present invention refers to multiple emulsions, having water as external phase, and having an improved water-repellence, comprising bifunctional derivatives (per)fluoropolyether as emulsifiers.

As known, the emulsions are dispersed systems of two immiscible liquids, one being of water and the other of one or more oils, in the presence of emulsifiers.

It is well known that emulsions are obtained wherein the dispersed phase is an oil and the continuous phase is composed of water, when in the emulsifier the hydrophilic portion is preponderant. In this way O/A emulsions are obtained wherein O represents the oil phase and A the water phase.

Emulsions are obtained wherein the dispersed phase is water and the continuous phase is formed of oil when in the emulsifier molecule the hydrophobic portion is preponderant. In this way A/O emulsions are obtained.

The O/A emulsions are those most commonly used for cosmetic applications as they are pleasant to the touch, i.e. they do not show oiliness characteristics. Besides the shelf life of these emulsions is rather good. However the O/A emulsions show the drawback to be easily removed from the skin by contact with water or also by the only skin perspiration. A further drawback of the O/A emulsions resides in that the water-repellent action is not very high. Therefore the protective action towards the skin irritating agents, soluble in water, is not satisfactory.

The A/O emulsions have a greater protective efficacy for the skin as they show a higher water-repellence. However these A/O emulsions are rarely used for cosmetic applications since they are somewhat oily. This drawback limits their use. Furthermore the A/O emulsions make much more difficult the skin perspiration, in particular in the temperate-warm climate countries. This phenomenon is further worsened by the presence of a high humidity.

Another drawback of O/A and A/O emulsions resides in that the emulsifier concentrations can also be high, for example they can even reach 12% by weight on the total of the composition. High emulsifier concentrations can imply negative effects for the local tolerability of these formulations. It is indeed well known that emulsifiers at high concentrations can cause skin irritation. This is due to the emulsifier as such, or to the fact that the compound makes easier the penetration of the irritating agent in the skin. A further drawback of the use of high amounts of emulsifiers is that there is a decrease of water-repellence.

Besides it is well known to add rheological modifiers, for example thixotropic agents, to increase the shelf life of O/A and A/O emulsions. These agents are solubilized or dispersed in the continuous phase of the emulsion. In particular, for O/A emulsions, as thixotropic agents, polymeric substances having hydrophilic characteristics, for example cellulosic polymers, are used. The drawback of these rheological modifiers is that they decrease the emulsion water-repellence. The negative effect of the emulsifiers and thixotropic agents is considerable, in particular for O/A emulsions, generally, not having, as said, a high water-repellence.

For the preparation of O/A and A/O emulsions see for example USP 4,803,067 describing emulsions for cosmetic use comprising (per)fluoropolyethers having non reactive end groups, with the function to confer water- and oleorepellence. The examples of this patent show that to obtain emulsions in the form of creams for cosmetic use, high amounts of emulsifying and thixotropic agents must be used. This, as said, is disadvantageous for the emulsion water-repellence. Furthermore tests carried out by the Applicant have shown that O/A emulsions comprising not reactive perfluoropolyethers have a poor water-repellence.

It is also known that in the publication of G. Pantini "Protective and sunscreen emulsions containing a perfluoropolyether amide" SOFW-Journal 127, 5-2001, pages 1-4, cosmetic compositions under the form of O/A emulsion containing a bifunctional perfluoropolyether derivative of alkylamide type, wherein the alkyl is linear C₁₈, are described. This composition shows an improved water-repellence in comparison with that obtained by using neutral perfluoropolyethers (having non reactive end groups). However the water-repellence values are still not very high.

The need was felt to have available emulsions having oleorepellence and an improved water-repellence, combined with a reduced oiliness, with respect to the prior art emulsions.

The Applicant has surprisingly and unexpectedly found compositions capable to solve the above technical problem.

An object of the present invention are compositions in the form of multiple emulsions having a X/O/A structure, wherein X or internal phase is a hydrophilic liquid immiscible with oil; O is an oily phase; A, also said external phase or continuous phase, is a water phase, said compositions comprising the following components:
A1) from 0.01% to 10% by weight, preferably from 0.1% to 5% by weight, still more preferably from 0.5% to 3% by weight of a bifunctional alkylamide (per)fluoropolyether derivative having formula:

   R' -NH-C (O) -CF₂O-R_{f}-CF₂-C (O) -NH-R (I)

   wherein
   - R and R', equal or different, are a C₁₃-C₂₀, preferably C₁₄-C₁₈, linear or branched alkyl chain;
   - R_{f} represents a (per)fluoropolyether chain comprising repeating units selected from one or more of the following:
      a)

         -(C₃F₆O)-;
      b)

         -(CF₂CF₂O)-;
      c)

         -(CFL₀O)-, wherein L₀ = -F, -CF₃;
      d)

         -CF₂(CF₂)_{z'}CF₂O-, wherein z' is an integer 1 or 2;
      e)

         -CH₂CF₂CF₂O-;
   the number average molecular weight of R_{f} being from 500 to 5,000, preferably from 1,000 to 2,500;
B1) from 0.1% to 0.5% by weight of a copolymer comprising monomers of acrylic type, swelling in water, **characterized by** the presence of acid groups and hydrophobic groups, having emulsifying activity after neutralization of the acid groups with a base; the pH of the composition being, at least, 5 and preferably not higher than 7.5;
   being 100% the sum of all the components, comprising the X, O and A phases and the substances dissolved and/or dispersed therein.

The oily phase constitutes, in per cent by weight, from 5% to 40%, preferably from 10% to 30%, of the composition according to the present invention.

The oily phase O comprises esters of C₄-C₁₂ monofunctional alcohols, or C₂-C₆ glycols or C₃-C₆ polyols, and C₈-C₃₀, hydrogenated or not, linear or branched, fat acids, or their mixtures. Examples of mixtures of the above esters are for example hydrogenated or not, vegetable oils.

The oily phase can be constituted by mixtures of said fat acid esters together with other additional oils selected from at least one of the following groups: mineral oils; linear or branched hydrocarbon oils preferably containing from 14 to 36 carbon atoms; liquid fluorinated polymers selected from perfluoropolyethers, dihydrofluoroethers and perfluoroalkanes.

The amount of the above additional oils is not higher than 50% by weight with respect to the total weight of the oily phase.

The A phase, or external phase, is generally a water solution containing additives soluble or dispersible in water, as for example cationic, anionic, nonionic emulsifiers in amounts from 0.01% to 5% by weight on the total of the composition; hydrophilic rheological modifiers, for example xanthan and carbomer rubber, in a concentration between 0.01% and 1% by weight on the total of the composition; and other additives, in total concentration in per cent by weight on the composition from 0.001% to 10%, preferably from 0.01% to 1%, of one or more of the following compounds: antioxidants, sequestrants; preservatives; perfumes; dyes; mineral powders; pigments; micronized solid polymers; sun screen as for example titanium dioxide and zinc oxide, hydrating agents as glycerine, diglycerine, propylene glycol, sugars, etc.

The X phase, called, as said, internal phase, is a hydrophilic liquid and can be water or a water solution, or a hydrophilic liquid different from water.

When the internal phase is a hydrophilic liquid in the form of a water solution, it can contain the same optional additives mentioned above for the phase A. In this case the amount of said additives, with respect to the composition weight, is within the above limits.

In the X phase also the following additives can optionally be contained: polyphenols, for example extracted from the grape seed or from green tea; C₂-C₆ hydroxyacids as, for example, glycolic acid, lactic acid, citric acid, tartatic acid; aromatic hydroxyacids such as for example salicylic acid, pyrrolidon carboxylic acid, proteic hydrolyzates, hyaluronic acid, aminoacids.

When X is a hydrophilic liquid different from water, it is preferably selected from C₂-C₁₀ linear or branched glycols or polyglycols; C₂-C₄ linear or branched monoalkylether glycols wherein the alkyl group is C₁-C₄; dimethoxymethane, known as methylal; polyalkylenglycols, preferably selected from polyoxyethylenglycol, preferably having number average molecular weight lower than 1,000, polyoxypropylenglycol having molecular weight lower than 2,000, polyoxyethylen-polyoxypropylen glycols having molecular weight lower than 3,000. Specific examples of hydrophilic liquids different from water are the following: ethylene glycol, methoxyethanol, propylene glycol, propan-1,2-diol, glycerine, diglycerine, dihydroxyacetone, methoxy-isopropanol, diethylene glycol, butan-1,4-diol, monoethylenether diethylenglycol, pentan-1,2-diol, monoethylether diethylenglycol, dipropylenglycol, monomethylether dipropylenglycol, monoethylether dipropylenglycol, still more preferably propylene glycol, glycerine and diglycerine.

The X and/or O phases can also optionally contain active principles as, for example, vitamins, for example vitamin C and vitamin E; ceramides; essential oils, and/or pharmaceutical active principles for topical use, as, for example, antiinflammatory agents, vasodilators, steroids, antibacterial compounds.

Said active principles are dissolved in the X phase or in the O phase, on the basis of their solubility, i.e. if soluble in water or in oil.

The whole amount of the phases X + O in per cent by weight on the total of the composition, ranges from 10% to 90%, preferably from 20% to 70%.

In R_{f} of component Al) the repeating unit -(C₃F₆O)- is selected between (CF(CF₃)CF₂O)- and/or -(CF₂-CF(CF₃)O)-

The (per)fluoropolyether chain R_{f} is preferably selected from the following structures:
1)

   -(CF₂O)ₐ-(CF₂CF₂O)_{b}-

   with b/a between 0.3 and 10, extremes included, a being an integer different from 0;
2)

   -(CF₂-(CF₂)_{z'}-CF₂O)_{b'}-

   wherein z' is an integer equal to 1 or 2;
3)

   -(C₃F₆O)ᵣ- (C₂F₄O)_{b}- (CFL₀O) ₜ-

   with r/b = 0.5-2.0, (r+b)/t = 10-30, b and t being integers different from 0;
4)

   -(OC₃F₆)ᵣ- (CFL₀O)ₜ-OCF₂-R'_{f}-CF₂O- (C₃F₆O)ᵣ- (CFL₀O)ₜ-
5)

   -(CF₂CF₂CH₂O)_{q'}-R'_{f}-O- (CH₂CF₂CF₂O)_{q'}-

   wherein:
   R'_{f} is a fluoroalkylene group from 1 to 4 carbon atoms; L₀ is selected between F, CF₃;
6)

   -(C₃F₆O)ᵣ-OCF₂-R'_{f}-CF₂O- (C₃F₆O)ᵣ-;
a, b, b',q', r, t, are integers, the sum of which is such that the (per) fluoropolyether chain R_{f} has number average molecular weight Mn values between 500 and 5,000 and preferably between 800 and 2,500.

The preferred (per)fluoropolyether chain R_{f} is selected from the following structures:

-(CF₂O)ₐ- (CF₂CF₂O)_{b}- ;

-(C₃F₆O)ᵣ- (C₂F₄O)_{b}-(CFL₀O) ₜ-;

wherein Lₒ and the a, b, r, t indexes have the above value; still more preferably R_{f} has the following structure:

-(CF₂O)ₐ-(CF₂CF₂O)_{b}-

Component B1) used as emulsifier is a copolymer comprising monomers of acrylic type, obtainable by copolymerization of a monoolefinic carboxylic monomer, preferably acrylic acid, with an ester of an acrylic acid with a C₈-C₃₀ alcohol.

Generally the amount of monoolefinic carboxylic monomer ranges from 40 to 99% by weight, preferably from 50 to 98%, and still more preferably from 80 to 98%. Monomeric mixtures of one or more monoolefinic carboxylic monomers with one or more esters of acrylic acids with C₈-C₃₀ alcohols can be used. Optionally the polymer can also be crosslinked by introducing in the monomeric mixture a crosslinking agent, generally in amounts between 0.1 and 4% by weight, preferably 0.2 and 1%, said amounts referred to the sum of the monomers in polymerization. Generally the crosslinking agent is a polymerizable monomer containing two or more double bonds. The carboxylic monomer generally contains from 3 to 6 carbon atoms. The preferred carboxylic monomers are of acrylic type having general formula:

CH₂=CR_{A}-COOH

wherein R_{A} is selected from H, halogen, OH, lactone, lactam, CN, C₁- C₂₀, preferably C₁-C₆, monovalent radicals, said radicals selected from the aryl, arylalkyl or alkylaryl, cycloaliphatic radicals. The acrylic acid is preferred.

The preferred acrylic esters are the alkylacrylates of formula:

CH₂=CR₁ - COOR₂

wherein R₁ is H, CH₃, C₂H₅; R₂ is an alkyl group from 8 to 30 carbon atoms, or an oxyalkylene, or carbonyloxyalkylene group. Among the oxyalkylene group, oxyethylene is preferred. Examples of acrylic esters are decyl (meth) acrylate, lauryl (meth) acrylate, stearyl(meth)acrylate, etc.

As crosslinking agents, polyalkenylpolyethers can be used.

Component B1) of the invention is prepared by polymerization in an inert solvent wherein the polymer is not soluble in a significant extent, or in a water medium. Among the solvents, benzene, xylene, hexane, ethyl acetate, etc., can be mentioned.

As polymerization initiators, ammonium or alkaline metal persulphates, benzoyl peroxide, cumene hydroperoxide, azoisobutyronitrile, redox catalysts can be mentioned.

The obtaind polymers are neutralized with bases to obtain the corresponding salts. Among the bases, alkales and substituted amines can be mentioned.

The emulsifiers on trade Pemulen^{®} TR by Noveon^{®}, in particular Pemulen^{®} TR-1 and Pemulen^{®} TR-2 are preferred; they are copolymers based on acrylic acid and acrylic esters with a C₁₀-C₃₀ alkyl chain.

The X/O/A emulsions of the present invention are generally prepared with a process comprising the following steps:
- heating of the phase X, wherein additives and active principles soluble in said hydrophilic liquid have previously been optionally dissolved at a temperature from 60°C to 100°C preferably from 80°C to 100°C;
- preparation of the oily phase O by solubilization at a temperature between 50°C and 60°C of the bifunctional alkylamide (per)fluoropolyether derivatives of formula (I) component A1), and optional additives and/or active principles soluble in said phase, in the esters of C₄-C₁₂ monofunctional alcohols, or C₂-C₆ glycols or C₃-C₆ polyols, with C₈-C₃₀ fat acids, optionally in admixture with the additional oils as above mentioned;
- heating of the oily phase O at a temperature in the range 70°C-90°C, preferably 80°C-90°C;
- adding the phase X to the oily phase O by operating with a stirring between 1,000 and 10,000 rpm, preferably between 1,000 rpm and 3,000 rpm for 10-20 minutes and obtainment of the primary emulsion X/O;
- cooling of the primary emulsion (emulsion X/O), under slow stirring, for example by operating with a stirring lower than 100 rpm, until the emulsion temperature is between 20°C and 60°C, preferably between 30°C and 50°C;
- dispersion of the copolymer component B1) in the external water phase A with the above mentioned optional excipients and subsequent addition of the primary emulsion X/O to the phase A, with a stirring of at least 1,000 rpm, to obtain a dispersion or diluted composition wherein the percentage by weight of primary emulsion is in the range 10%-90%, preferably 30%-60%; this step generally requires a few minutes, generally no more than 5 minutes;
- addition to the composition of an aqueous base, for example sodium hdyroxide, to obtain a pH value between 5.0 and 7.5, preferably 5.5-7.

Surprisingly and unexpectedly, the formulations of the present invention allow to obtain an improved water-repellent activity, and generally a higher viscosity (see the Examples), the composition being equal to that of the corresponding fromulations O/A.

It has been found by the Applicant that to obtain O/A emulsions in the form of creams having viscosity comparable with those of the formulations of the present invention, the content of component A1) being equal, it is necessary to add thixotropic agents. Besides, the water-repellent properties of these formulations O/A additioned of said thixotropic agents are not satisfactory. See the comparative Examples.

The formulations according to the present invention, differently from the O/A emulsions, even containing reduced amounts of emulsifiers and viscosifiers, allow to obtain formulations having also high viscosity and therefore in these cases the addition of hydrophilic thixotropic agents is not required. It is thus possible to prepare creams having a high water-repellent activity and therefore improved skin protection.

Furthermore the compositions of the present invention are stable to storage and have high shelf life; for example they are stable for at least 6 months.

The compositions of the present invention have an improved local tolerability and an improved persistence on the skin in comparison with the O/A formulations, even with the same composition.

As said, the compositions of the present invention are usable to prepare cosmetic formulations.

The compositions of the present invention can be used as such when a high skin protection and high water-repellence are required.

Furthermore the viscosity of the compositions according to the present invention can be adjusted without adding thixotropic agents or other additives, but varying the concentrations of components A1) and/or B1) and/or the amount of the hydrophilic liquid X.

It is therefore possible to obtain compositions having a suitable viscosity for all uses in the field of the skin topical applications.

With the compositions of the present invention it is thus possible to prepare formulations for the following uses: spray applications, wherein a viscosity lower than 1,000 mPa.s is required; dispenser for applications of compositions having a viscosity generally higher than 1,000 mPa.s and not higher than 15,000 mPa.s; emulsions generally having viscosity from 15,000 mPa.s and up to 30,000 mPa.s, usable from vessels, creams in tubes or in little pots, in the case of preparations in emulsion having a very high viscosity.

The compositions of the present invention can have therefore the most varied uses, on the basis of the additives and/or the active principles contained therein.

It is possible to prepare cosmetic formulations, for example formulations for the scalp, for example lotions, dyes, and formulations for the face or the skin protection in general.

As said, the formulations of the present invention can also be used as bases to prepare pharmaceutical compositions for topical use and therefore they are a carrier for therapeutic active principles.

Furthermore it has been found by the Applicant that when the active principle or the active principles used in the composition are potentially irritating for the skin, and they are dispersed or solubilized in the internal phase X, with the compositions of the present invention a very gradual release to the skin of said active principles is obtained and therefore the irritating potential of the compounds is significantly reduced.

The following non limitative Examples illustrate the invention.

### EXAMPLES

### Methods

### Identification test of the emulsion type or of the continuous phase of the emulsion

A small amount of the emulsion to be tested is dispersed in a beaker containing water (or a hydrophilic liquid), by manually stirring. If there is a rapid dispersion in water, it means that the continuous phase or external phase of the emulsion is water and that the emulsion is an O/A emulsion. If, on the contrary, there is no dispersion, the continuous phase or external phase of the emulsion is an oil and the emulsion to be tested is of the A/O type. While repeating the test with the A/O emulsion but by using an oil, it is noticed that the emulsion rapidly disperses.

### Emulsion stability test

The emulsion is centrifuged at 5,000 rpm for 20 minutes. The emulsion is in compliance with the test, therefore it is stable according to the test, if there is no segregation, i.e. the oil rises on the surface and/or the water separates on the bottom of the vessel.

### Viscosity determination

The viscosity is determined at the temperature of 25°C in a Brookfield viscometer, at the revolution number and with rotor as specified in the Examples.

### Water-repellence test

About 0.5 ml of the composition to be tested are applied on a filter paper having the following characteristics: porosity 17÷25 *µ*m, and density 75 g/m² (Carlo Erba Reagenti, Milano) delimiting with a pencil the zone resulting wet. It is let dry for 20 minutes and then a drop of water is applied on the treated zone, and, as reference, on a zone of the filter paper not treated. The paper absorption time is determined. In the case of the untreated zone, it is noticed that the absorption is practically instantaneous.

### Test for the evaluation of the resistance of the invention compositions to the removal with water

The following test has been used. The lower part of each of the two forearms of No. 4 volunteers is depilated so as to have a zone with a 50 cm² area. The so delimited zone is carefully cleaned with soap and then with cotton-wool soaked in ethyl alcohol. 100 mg of the composition are spread on the surface located on the right forearm of each volunteer. The corresponding zone on the left forearm is not treated and acts as reference control. The formulation is let dry on the skin for 20 minutes and then the forearms are immersed for 20 minutes in water at the temperature of 25°C. The water is then removed, it is let dry for 20 minutes and then the forearm immersion is repeated again as above described. At the end it is let dry again for 20 minutes and the residual composition is removed with cotton-wool soaked in ethyl alcohol, repeating the operation also on the untreated forearm, so as to have a reference control. The alcoholic phases are dried under vacuum and it is respectively brought to the volume of 100 ml with ethyl alcohol. The specimen absorbance is read in the UV field against the blank. The absorbance average value is calculated. The obtained value is reported on a calibration straight line having in ordinates the absorbance of octylmethoxycinnamate and in abscissae the corresponding concentration of the compound in ethanol, expressed in mcg/ml. From the straight line the concentration is extrapolated and one attains to the average total amount of the compound remained on the forearms by multiplying the obtained value by 100. It is calculated the ratio between the compound amount, recovered, with respect to that present in the same amount (100 mg) of formulation.

### EXAMPLE 1

Preparation of a multiple emulsion by using the perfluoropolyether PFPE di-stearylamide derivative (Fomblin^{®}SA/18) having the following formula:

C₁₈H₃₇-NH-C(O)-CF₂O-(CF₂O)ₐ-(CF₂CF₂O)_{b}-CF₂-C(O) -NH-C₁₈H₃₇

wherein b/a = 0.5÷3.0, and the number average molecular weight of the perfluoropolyether chain is 1,400.

A multiple emulsion was obtained with the following process:
A) An emulsion of the water-in-oil (A/O) type, called primary, is prepared, containing the perfluoropolyether derivative, emollient oils and water, mixing as indicated hereunder the emulsion compounds (the percentages are by weight, between brackets the percentage by weight of the same compound calculated on the final multiple emulsion) :
- PFPE di-stearylamide 2.5 (1.5)
- Mineral oil 11.7 (7.0)
- Octylpalmitate 13.3 (8.0)
- Caprylic/capric triglyceride 13.3 (8.0)
- Water 59.2 (35.5)
The oil phase of the primary emulsion is prepared by heating the oils at a temperature higher than 50°C, so as to melt and solubilize PFPE di-stearylamide, then the oily solution is heated to 80°C; the water to be added to the emulsion is previously heated at 90°C and then slowly added to the oily phase, under strong stirring, in a turbomixer operating at 1,500-3,000 revolutions per minute, maintaining the stirring for a total of 20 minutes. At the end the speed is decreased under 100 rev/min letting cool the emulsion up to 40-45°C.
B) Preparation multiple emulsion water-oil-water (A/O/A) (the percentages are in per cent by weight):
- Primary emulsion A) 60.0
- Acrylic copolymer hydrophobically modified with C₁₀₋₃₀ alkyl chains (Pemulen^{®} TR-1, Noveon Inc.) 0.2
- Sodium hydroxide 0.05
- Preservative (phenoxyethanol/parabens) 0.5
- Water 39.25

| | |
|---|---|
| pH 6.4 | |
| Recognition test continuous phase: | water |
| Stability test: | conformable |
| Viscosity (10 rpm, rotor 29): | 35,500 mPa.s |
| Water-repellence test: | > 2 hours |

The multiple emulsion is obtained by first preparing a water phase, dispersing the Pemulen^{®} TR-1 at a temperature comprised between 30°C and 45°C, under stirring, in most of the water amount indicated in B). To the obtained dispersion it is added, under strong stirring (1,500-3,000 rev/min), for some minutes, for example 2-5 minutes, the amount of the primary emulsion A/O indicated in B) to the Pemulen^{®} dispersion. Then it is added, under slow stirring, the sodium hydroxide dissolved in the residual water and lastly the preservative. In this case the water is internal phase, as surrounded by the oil phase, and external phase as it forms the continuous phase, separated from the internal phase A by the oily phase.

### EXAMPLES 2 and 2A (comparative)

Two solid emulsions (creams) of the oil in water (O/A) type were prepared, so as to have water as continuous phase, as in the case of the multiple emulsion of the Example 1.

As emulsifying agent, a polymer of the acrylic acid hydrophobically modified (Pemulen^{®}TR-1) was used.

A tixotropic agent (hydrophilic polymer, named INCI Carbomer, on trade Carbopol^{®} Ultrez 10, Noveon Inc.) was added to obtain a viscosity of the same order of magnitude of the multiple emulsion of the Example 1.

The formulation of the Example 2A (comp.), differently from that of the Example 2 (comp.), contains the PFPE-di-stearylamide compound. The obtained compositions are reported hereinafter.

The percentages are by weight on the total of the composition:

| | Ex. 2 (comp.) | Ex. 2A (comp.) |
|---|---|---|
| | (%) | (%) |
| PFPE stearylamide | - | 1.5 |
| Mineral oil | 7.5 | 7.0 |
| Octylpalmitate | 8.5 | 8.0 |
| Caprylic/capric triglyceride | 8.5 | 8.0 |
| Water | 74.5 | 74.5 |
| Pemulen^{®}TR-1 | 0.2 | 0.2 |
| Carbopol^{®} Ultrez 10 | 0.2 | 0.2 |
| Sodium hydroxide | 0.1 | 0.1 |
| Preservatives (phenoxyethanol/parabens) | 0.5 | 0.5 |
| pH about | 6.5 | 6.5 |
| Recognition test emulsion type | O/A | O/A |
| Stability Test | conf.° | conf.° |
| Viscosity (10 rpm, rotor 29) (mPa.s) | 24,500 | 30,500 |
| Water-repellence Test | absorption* | 30 min. |

| | | |
|---|---|---|
| * immediate absorption ° conf = conformable | | |

The preparation of the emulsions of the Examples 2 and 2A (comp.) is carried out as follows. The oil phase is prepared by mixing the oils at room temperature (20°C-25°C). In the case of the Example 2A (comp.) the mixture is heated to 55°C before adding PFPE di-stearylamide. A water phase containing Pemuleri^{®}TR-1 and Carbopol^{®}Ultrez 10 is prepared by dispersing at room temperature said compounds in most part of the water, used to prepare each formulation. In the remaining water the sodium hydroxide is dissolved. To prepare the composition of the Example 2 (comp.), the oil phase is added to the water dispersion containing Pemulen TR-1 and Carbopol^{®}Ultrez under strong stirring (1,500-3,000 rev/minute), for some minutes (2-5 minutes); then under moderate stirring one neutralizes with sodium hydroxide up to pH between 5.5 and 6.5; at the end the preservatives are added.

In the case of the Example 2A (comp.) the oily phase, at the above temperature, is added to the water phase previously heated to 45°C, under strong stirring, in a turbomixer operating at 1,500-3,000 revolutions per minute, maintaining the stirring for 2-5 minutes. At the end the speed is decreased under 100 rev/min, letting cool up to 40°-45°C.

### Comments to the Examples 1 and 2A (comp.).

The Example 2A (comp.) shows that an O/A emulsion containing the same PFPE di-stearylamide amount as in the multiple A/O/A emulsion of the Example 1, must be additioned of a viscosifying agent to have a viscosity comparable with that of the Example according to the present invention. The Example 2A (comp.) shows that with the same content of PFPE di-stearylamide, the water-repellence of the O/W emulsion is remarkably lower than that of the multiple emulsion.

### EXAMPLE 3 (comparative)

The Example 1 is repeated, by using in the preparation of the primary emulsion A) PFPE di-laurylamide instead of PFPE di-stearylamide. It has been found that it is not possible to prepare the primary emulsion by operating under the same conditions of the Example 1. The emulsion can be prepared by adding A/O surfactants. The Applicant has verified that the water-repellence of the obtained multiple emulsion is anyhow clearly lower than that of the formulations of the present invention.

### EXAMPLE 4 (comparative)

The Example 1 is repeated, by using in the preparation of the multiple emulsion B) a surfactant formed of an ethylene oxide/propylene oxide block copolymer, m.p. 52°C, commercially known with the trademark Synperonic^{®}PE/F127, in an amount equal to 2% to obtain the formation of the multiple emulsion.

This emulsifier is commonly used to prepare multiple emulsions.

The results obtained in the above described tests are the following:

| | |
|---|---|
| pH | 6.5 |
| Recognition test continuous phase: | water |
| Stability test: | not conformable* |
| Viscosity (10 rpm, rotor 29): | 6,500 mPa.s |
| Water-repellence test | 20 minutes |

| | |
|---|---|
| * segregation of phases is observed (the emulsion breaks) | |

### Comment to the Example 4 (comparative)

The Example shows that by using a polymeric emulsifier commonly used to prepare multiple emulsions, but not having an acrylic polymeric structure as defined in the present invention, it is not possible to obtain the invention formulations since the obtained multiple emulsion is not stable and the water-repellence is unsatisfactory.

### EXAMPLE 5 (comparative)

An O/A emulsion is prepared with the same ingredients and in the same amount used for the preparation of the multiple emulsion of the Example 1.

The oily phase is prepared as described in the Example 1.

The aqueous dispersion of Pemulen^{®}TR-1 and the sodium hydroxide solution are prepared as described in the Example 1. Under strong stirring (1,500-3,000 rev/minute), in a Turbomixer, to the water phase containing Pemulen^{®}TR-1 the oily phase is added, it must have a temperature between 30°C and 40°C. Lastly it is cooled at room temperature (20°C-25°C).

When the addition is over, the stirring is slackened and the sodium hydroxide solution is slowly poured into the mixture.

The obtained results in the above tests are the following:

| | |
|---|---|
| pH | 6.6 |
| Recognition test continuous phase: | water |
| Stability test: | conformable |
| Viscosity (10 rpm, rotor 29): | 6,500 mPa.s |
| Water-repellence test | 50 minutes |

### Comment to the Example 5 (comparative)

The Example 5 (comparative) shows that an O/A emulsion containing the same amount of active principle and of ingredients as the multiple emulsion of the Example 1, does not allow to obtain a water-repellence value comparable with that of the Example 1. Furthermore the viscosity is much lower than that of the preparation according to the invention.

### EXAMPLE 6 (comparative)

The Example 1 is repeated but by using in the preparation of the primary emulsion A) a PFPE having neutral (unreactive) end groups, having molecular weight 6,250, commercially known with the trademark Fomblin^{®}HCR. It has been found that by operating in the same conditions of the Example 1 it is not possible to prepare the primary emulsion. It is observed that, at rest, there is an immediate segregation.

### EXAMPLE 7 (comparative)

An emulsion of the O/A type, containing PFPE di-stearylamide and diglycerine was prepared, having the following composition, in per cent by weight:

| | (%) |
|---|---|
| PFPE di-stearylamide | 1.00 |
| Mineral oil | 6.00 |
| Octylpalmitate | 7.00 |
| Caprylic/capric triglyceride | 7.00 |
| diglycerine | 5.00 |
| Pemulen^{®}TR-1 | 0.20 |
| Sodium hydroxide | 0.06 |
| Preservatives (phenoxyethanol/parabens) | 0.50 |
| Water | 73.24 |
| pH about | 6.50 |
| Recognition test emulsion type | O/A |
| Stability test | conf.* |
| Viscosity (10 rpm, rotor 21) (mPa.s) | 1,200 |
| Water-repellence test | 30 min. |

| | |
|---|---|
| * conf. = conformable | |

### EXAMPLE 8

A multiple emulsion was prepared according to the present invention in the form of diglycerine/O/A having the same composition of the O/A emulsion of the Example 7 (comp.).

The primary emulsion used for the preparation of the complex emulsion had the following composition in per cent by weight (between brackets, the percentage by weight referred to the complex emulsion):

| | (%) |
|---|---|
| PFPE di-stearylamide | 3.9 (1.0) |
| Mineral oil | 23.1 (6.0) |
| Octylpalmitate | 26.9 (7.0) |
| Caprylic/capric triglyceride | 26.9 (7.0) |
| Diglycerine | 19.2 (5.0) |

The primary emulsion is prepared as described in the Example 1, by using diglycerine instead of water.

The multiple emulsion diglycerine-oil-water (diglycerine/O/A) is prepared by using the amounts of primary emulsion and of the other components mentioned hereunder (in per cent by weight):

| | |
|---|---|
| Primary emulsion | 26.00 |

- (Pemulen^{®}TR-1, Noveon Inc.) 0.20
- Sodium hydroxide 0.06
- Preservative (phenoxyethanol/parabens) 0.50
- Water 73.24

| | |
|---|---|
| pH about 6.4 | |
| Recognition test continuous phase | water |
| Stability test | Conformable |
| Viscosity (10 rpm, rotor 21) (mPa.s) | 2,200 |
| Water-repellence test | > 2 hours |

The multiple emulsion is prepared with the same process described in the Example 1.

### Comments to the Examples 7 (comp.) and 8 according to the invention.

These Examples show that by using as internal phase an aliphatic polyol as diglycerine, instead of water, a composition under the form of multiple emulsion is obtained having a high water-repellence value with respect to the O/A emulsion having the same composition. Besides also the viscosity is about the double, the concentration of PFPE di-stearylamide being equal, of that of the O/A emulsion.

### EXAMPLE 9

A multiple emulsion according to the present invention was prepared in the form of diglycerine/O/A modifying the ingredients so as to obtain an emulsion with suitable viscosity to be used from a tube vessel.

The primary emulsion used for the preparation of the complex emulsion had the following composition in per cent by wqeight (between brackets, the percentage by weight referred to the complex emulsion):

| | (%) |
|---|---|
| PFPE di-stearylamide | 4.6 (1.5) |
| Mineral oil | 21.6 (7.0) |
| Octylpalmitate | 24.6 (8.0) |
| Caprylic/capric triglyceride | 24.6 (8.0) |
| diglycerine | 24.6 (8.0) |

The primary emulsion is prepared as described in the Example 1 by using diglycerine instead of water.

The multiple emulsion diglycerine-oil-water (diglycerine/O/A) is prepared by using the amounts of primary emulsion and of the other components indicated hereunder (in per cent by weight) :

| | |
|---|---|
| Primary emulsion | 32.50 |

- Acrylic polymer partially esterified with C₁₀₋₃₀ alkyl chains (Pemulen^{®}TR-1,
   Noveon Inc.) 0.40
- Sodium hydroxide 0.12
- Preservative (phenoxyethanol/parabens) 0.60
- Water 66.38

| | |
|---|---|
| pH about 5.7 | |
| Recognition test continuous phase | water |
| Stability test | conformable |
| Viscosity (10 rpm, rotor 21) (mPa.s) | 30,000 |
| Water-repellence test | > 2 hours |

### EXAMPLE 10 (comparative)

An O/A emulsion of the same composition of the Example 2A (comp.) was prepared, but containing a perfluoropolyether having neutral (unreactive) end groups with molecular weight 3,200, commercially available with the trademark Fomblin^{®}HC25, instead of PFPE di-stearylamide.

The results obtained in the tests are the following:
pH about 6.45

| | |
|---|---|
| Recognition test emulsion type | O/A |
| Stability test | conf. |
| Viscosity (10 rpm, rotor 21) (mPa.s) | 29,500 |
| Water-repellence test | < 5 min |

### Comment to the Example 10 (comp.).

The Example shows that the water-repellent power of an O/A emulsion containing a perfluoropolyether having neutral (unreactive) end groups is lower than that of a similar O/A emulsion but containing PFPE di-stearylamide (Example 2A (comp.)).

### EXAMPLE 11

Preparation of a sun protective composition and evaluation of the resistance to the removal with water.

The process described in the Example 1 is followed.

A primary emulsion A) water-in-oil is first prepared having the following composition:
- PFPE di-stearylamide 3.0 (1.5)
- Mineral oil 17.0 (8.5)
- Octylpalmitate 18.0 (9.0)
- Caprylic/caprictriglyceride 13.3 (8.0)
- Octylmethoxycinnamate (UV-B filter) 10.0 (5.0)
- Methoxydibenzoylmethane (UV-A filter) 2.0 (1.0)
- Water 50.0 (25.0)

### B) Preparation multiple emulsion water-oil-water (A/O/A):

- Primary emulsion A) 50.0
- Acrylic copolymer hydrophobically modified with C₁₀₋₃₀ alkyl chains (Pemulen^{®}TR-1, Noveon Inc.) 0.4
- Sodium hydroxide 0.12
- Preservative (phenoxyethanol/parabens) 0.60
- Water 48.88

| | |
|---|---|
| pH 6.0 | |
| Recognition test continuous phase: | water |
| Stability test: | conformable |
| Viscosity (10 rpm, rotor 29) : | 26,000 mPa.s |
| Water-repellence test | > 2 hours |
| Resistance test to water | 95% |

## Claims

1. Compositions in the form of multiple emulsions having a X/O/A structure, wherein X or internal phase is a hydrophilic liquid immiscible with oil; O is an oily phase; A, external phase or continuous phase, is a water phase, said compositions comprising:
A1) from 0.01% to 10% by weight of a bifunctional alkylamide (per)fluoropolyether derivative having formula:
R' -NH-C(O) -CF₂O-R_{f}-CF₂-C(O) -NH-R (I)
wherein
- R and R', equal or different, are a C₁₃-C₂₀, linear or branched alkyl chain;
- R_{f} represents a (per)fluoropolyether chain comprising repeating units selected from one or more of the following:
a)
-(C₃F₆O)-;
b)
-(CF₂CF₂O)-;
c)
-(CFL₀O)-, wherein L₀ = -F, -CF₃;
d)
-CF₂(CF₂)_{z'}CF₂O-, wherein z' is an integer 1 or 2;
e)
-CH₂CF₂CF₂O-;
the number average molecular weight of R_{f} being from 500 to 5,000;
B1) from 0.1% to 0.5% by weight of a copolymer, comprising monomers of acrylic type, swelling in water, **characterized by** the presence of acid groups and hydrophobic groups, having emulsifying activity after neutralization of the acid groups with bases;
the pH of the composition being at least 5;
wherein the oil phase O constitutes from 5% to 40% and X+O ranges from 10% to 90% as per cent by weight on the total composition;
wherein the oily phase O, comprises esters of C₄-C₁₂ monofunctional alcohols, or C₂-C₆ glycols or C₃-C₆ polyols, with C₈-C₃₀, hydrogenated and not, linear or branched, fat acids, or their mixtures;
being 100% the sum of all the components, comprising the X, O and A phases and the substances dissolved and/or dispersed therein.

2. Compositions according to claim 1, wherein the oil phase constitutes, in per cent by weight, from 10% to 30%.

3. Compositions according to claims 1-2, wherein the oily phase O comprises mineral oils; linear or branched hydrocarbon oils preferably containing from 14 to 36 carbon atoms; liquid fluorinated polymers selected from perfluoropolyethers, dihydrofluoroethers and perfluoroalkanes, the amount of said oils being not higher than 50% by weight with respect to the total weight of the oily phase.

4. Compositions according to claims 1-3, wherein the phase A optionally contains additives soluble or dispersible in water, preferably selected from cationic, anionic, nonionic emulsifiers; hydrophilic rheological modifiers, antioxidants; sequestrants; perfumes; preservatives; dyes; mineral powders; pigments; micronized solid polymers; sun filters; hydrating agents.

5. Compositions according to claims 1-4, wherein the X phase is water or a water solution or a hydrophilic liquid different from water.

6. Compositions according to claim 5, wherein the X phase is a water solution and contains additives soluble or dispersible in water, selected from cationic, anionic, nonionic emulsifiers; hydrophilic rheological modifiers, antioxidants; sequestrants; perfumes; preservatives; dyes; mineral powders; pigments; micronized solid polymers; sun filters; hydrating agents.

7. Compositions according to claims 5-6, wherein the X phase contains the following additives: polyphenols, C₂-C₆ hydroxyacids; aromatic hydroxyacids preferably salicylic acid; pyrrolidon carboxylic acid; proteic hydrolyzates; hyaluronic acid; aminoacids.

8. Compositions according to claim 5, wherein the X phase is a hydrophilic liquid different from water selected from C₂-C₁₀ linear or branched glycols or polyglycols; C₂-C₄ linear or branched monoalkylether glycols wherein the alkyl group is C₁-C₄; dimethoxymethane; polyalkylen-glycols selected from polyoxyethylenglycols having number average molecular weight lower than 1,000; polyoxy-propylenglycols having molecular weight lower than 2,000; polyoxyethylenpolyoxypropylen glycols having molecular weight lower than 3,000.

9. Compositions according to claims 1-8, wherein the X and/or O phases contain pharmaceutical active principles for topical use.

10. Compositions according to claims 1-9, wherein the total amount of the phases X + O, expressed in per cent by weight on the total of the composition, ranges from 20% to 70%.

11. Compositions according to claims 1-10, wherein in R_{f} of the component A1) the repeating unit (C₃F₆O) - is selected between - (CF(CF₃)CF₂O) - and/or - (CF₂-CF(CF₃)O)-.

12. Compositions according to claims 1-11, wherein the (per) fluoropolyether chain R_{f} is selected from the following structures:
1)
-(CF₂O)ₐ-(CF₂CF₂O)_{b}-
with b/a between 0.3 and 10, extremes included, a being an integer different from 0;
2)
-(CF₂-(CF₂)_{z'}-CF₂O)_{b'}-
wherein z' is an integer equal to 1 or 2;
3)
-(C₃F₆O)ᵣ-(C₂F₄O)_{b}-(CFL₀O)ₜ-
with r/b = 0.5-2.0, (r+b)/t = 10-30, b and t being integers different from 0;
4)
- (OC₃F₆)ᵣ-(CFL₀O)ₜ-OCF₂-R'_{f}-CF₂O-(C₃F₆O)ᵣ-(CFL₀O)ₜ-;
5)
-(CF₂CF₂CH₂O)_{q'} -R'_{f}-O- (CH₂CF₂CF₂O)_{q'}-
wherein:
R'_{f} is a fluoroalkylene group from 1 to 4 carbon atoms;
L₀ is selected between F, CF₃;
6)
-(C₃F₆O)ᵣ-OCF₂-R'_{f}-CF₂O- (C₃F₆O) ᵣ-;
a, b, b',q', r, t, being integers, the sum of which is such that the (per)fluoropolyether chain R_{f} has number average molecular weight Mn values between 500 and 5,000.

13. Compositions according to claim 12, wherein the (per) fluoropolyether chain R_{f} is selected from the following structures:
- (CF₂O)ₐ-(CF₂CF₂O)_{b}-;
- (C₃F₆O)ᵣ- (C₂F₄O)_{b}- (CFL₀O)ₜ-;
wherein L₀ and the a, b, r, t indexes have the above value.

14. Compositions according to claims 1-13, wherein the component B1) is a copolymer comprising monomers of acrylic type, obtainable by copolymerization of a monoolefinic carboxylic monomer with an ester of an acrylic acid with a C₈-C₃₀ alcohol.

15. Compositions according to claim 14, wherein the carboxylic monomer of acrylic type has general formula:
CH₂=CR_{A}-COOH
wherein R_{A} is selected from H, halogen, OH, lactone, lactam, CN, C₁-C₂₀, monovalent radicals, said radicals selected from aryl, arylalkyl or alkyl-aryl, cycloaliphatic radicals.

16. Compositions according to claims 14-15, wherein the acrylic esters have formula:
CH₂=CR₁ - COOR₂
wherein R₁ is H, CH₃, C₂H₅; R₂ is an alkyl group from 8 to 30 carbon atoms, or oxyalkylene, or carbonyloxyalkylene group.

17. Compositions according to claims 14-16, wherein the copolymers are based on acrylic acid and acrylic esters with a C₁₀-C₃₀ alkyl chain.

18. A process to obtain the X/O/A emulsions of claims 1-17 comprising the following steps:
- heating of the phase X, wherein additives and active principles soluble in said hydrophilic liquid have previously been optionally dissolved at a temperature from 60°C to 100°C;
- preparation of the phase O by solubilization at a temperature between 50°C and 60°C of the bifunctional alkylamide (per) fluoropolyether derivatives of formula (I) component A1), and optionally possible additives and/or active principles soluble in said phase, in the esters of C₄-C₁₂ monofunctional alcohols, C₂-C₆ glycols or C₃-C₆ polyols, with C₈- C₃₀ fat acids, optionally in admixture with other oils;
- heating of the phase O at a temperature in the range 70°C-90°C;
- adding the phase X to the phase O under a stirring between 1,000 and 10,000 rpm for 10-20 minutes and obtainment of the primary emulsion X/O;
- cooling of the primary emulsion X/O until the emulsion temperatures is between 20°C and 60°C;
- dispersion of the copolymer component B1) in the external water phase A with optional excipients and subsequent addition of the X/O emulsion to the phase A, with a stirring of at least 1,000 rpm, obtaining a dispersion wherein the percentage by weight of primary emulsion is in the range 10%-90%;
- addition to the final composition of an aqueous base to obtain a pH value between 5.0 and 7.5.

19. Use of the compositions of claims 1-17 to prepare cosmetic compositions having water-repellent activity.

20. Use of the compositions of claims 1-17 as bases to prepare pharmaceutical compositions for topical use.

21. Compositions according to claim 1 wherein A1) is from 0.1% to 5% by weight, R and R', equal or different, are C₁₄-C₁₈, linear or branched alkyl chain.

22. Compositions according to claim 1 wherein the number average molecular weight of R_{f} of A1) is from 1,000 to 2,500.

23. Compositions according to claim 1 wherein the pH of the composition is not higher than 7.5.

24. Compositions according to claim 21 wherein A1) is from 0.5% to 3% by weight.

25. Compositions according to claim 1-8, wherein the X and/or O phases contain active principles selected from the group of vitamin C and vitamin E; ceramides; essential oils.

26. Compositions according to claim 13, wherein the (per) fluoropolyether chain R_{f} is - (CF₂O)ₐ- (CF₂CF₂O)_{b}-, wherein a and b have the meaning indicated above.

27. Compositions according to claim 14, wherein the monoolefinic carboxylic monomer is acrylic acid.

28. Compositions according to claim 15 wherein R_{A} is a C₁- C₆ monovalent radical.

29. A process according to claim 18 wherein heating of the phase O is performed at a temperature in the range 80°C-90°C.

30. A process according to claim 18 wherein in the dispersion obtained by adding the X/O emulsion to the phase A the percentage by weight bf primary emulsion is in the range 30% - 60%.

31. A process according to claim 18 wherein after addition of the aqueous base to the final dispersion the pH is in the range 5.5-7.

32. Compositions according to claims 1-8 containing the active principles of claim 25 and pharmaceutical active principles for topical use.

## Patentansprüche

1. Zusammensetzungen in Form von Mehrfach-Emulsionen mit einer X/O/A-Struktur, wobei X oder innere Phase eine mit Öl nicht mischbare, hydrophile Flüssigkeit ist; O eine Ölphase ist, A, die äußere Phase oder Dispersionsphase, eine Wasserphase ist, wobei die Zusammensetzungen umfassen:
A1) 0,01 Gew.-% bis 10 Gew.-% eines bifunktionellen Alkylamid(per)fluorpolyetherderivats der Formel
R'-NH-C(O)-CF₂O-R_{f}-CF₂-C(O)-NH-R (I)
wobei
- R und R', gleich oder verschieden, eine geradkettige oder verzweigte C₁₃-C₂₀-Alkylkette sind;
- R_{f} eine (Per)fluorpolyetherkette darstellt, umfassend Wiederholungseinheiten, ausgewählt aus einer oder mehreren der folgenden:
a)
-(C₃F₆O)-;
b)
-(CF₂CF₂O)-;
c)
-(CFL₀O)-, wobei L₀ = F, -CF₃
ist;
d)
-CF₂(CF₂)_{z'}CF₂O- ,
wobei z' eine ganze Zahl von 1 oder 2 ist;
e)
-CH₂CF₂CF₂O-;
wobei das zahlenmittlere Molekulargewicht von R_{f} 500 bis 5000 beträgt;
B1) 0,1 Gew.-% bis 0,5 Gew.-% eines Copolymers, umfassend Monomere vom Acryltyp, in Wasser quellend, **gekennzeichnet durch** die Gegenwart von Säureresten und hydrophoben Resten, mit Emulsionsaktivität nach Neutralisierung der Säurereste mit Basen;
wobei der pH-Wert der Zusammensetzung mindestens 5 beträgt;
wobei die Ölphase O 5 % bis 40 % ausmacht und X + O im Bereich von 10% bis 90% liegt, als Gewichtsprozent der gesamten Zusammensetzung;
wobei die Ölphase O Ester monofunktioneller C₄-C₁₂-Alkohole oder C₂-C₆-Glycole oder C₃-C₆-Polyole mit hydrierten oder nichthydrierten, geradkettigen oder verzweigten C₈-C₃₀-Fettsäuren, oder deren Gemische umfasst;
wobei 100% die Summe aller Bestandteile ist, umfassend die X-, O- und A-Phasen und die Substanzen, die darin gelöst und/oder dispergiert sind.

2. Zusammensetzungen gemäß Anspruch 1, wobei die Ölphase 10 bis 30 Gew.-% ausmacht.

3. Zusammensetzungen gemäß den Ansprüchen 1 - 2, wobei die Ölphase O Mineralöle umfasst; geradkettige oder verzweigte Kohlenwasserstofföle, welche vorzugsweise 14 bis 36 Kohlenstoffatome enthalten, flüssige fluorierte Polymere, ausgewählt aus Perfluorpolyethern, Dihydrofluorethern und Perfluoralkanen, umfasst; und wobei die Menge dieser Öle nicht mehr als 50 Gew.-%, bezogen auf das Gesamtgewicht der Ölphase, beträgt.

4. Zusammensetzungen gemäß den Ansprüchen 1 - 3, wobei die Phase A gegebenenfalls Additive enthält, welche in Wasser löslich oder dispergierbar sind, vorzugsweise ausgewählt aus kationischen, anionischen, nicht ionischen Emulgatoren; hydrophilen Rheologie-Modifikatoren, Antioxidantien; Komplexbildnern; Parfümen, Konservierungsmitteln; Farbstoffen; Mineralpulvern; Pigmenten; mikronsierten Festpolymeren; Sonnenfiltern; Hydrierungsmitteln.

5. Zusammensetzungen gemäß den Ansprüchen 1-4, wobei die X-Phase Wasser oder eine Wasserlösung oder eine von Wasser verschiedene hydrophile Flüssigkeit ist.

6. Zusammensetzung gemäß Anspruch 5, wobei die X-Phase eine Wasserlösung ist und Additive enthält, welche in Wasser löslich oder dispergierbar sind, ausgewählt aus kationischen, anionischen, nicht ionischen Emulgatoren; hydrophilen Rheologie-Modifikatoren, Antioxidantien; Komplexbildnern; Parfümen; Konservierungsmitteln; Farbstoffen; Mineralpulvern; Pigmenten; mikronsierten Festpolymeren; Sonnenfiltern; Hydrierungsmitteln.

7. Zusammensetzungen gemäß den Ansprüchen 5 - 6, wobei die X-Phase die folgenden Additive enthält: Polyphenole, C₂-C₆-Hydroxysäuren; aromatische Hydroxysäuren, vorzugsweise Salicylsäure; Pyrrolidoncarbonsäure; Proteinhydrolysate; Hyaluronsäure; Aminosäuren.

8. Zusammensetzungen gemäß Anspruch 5, wobei die X-Phase eine von Wasser verschiedene hydrophile Flüssigkeit ist, ausgewählt aus geradkettigen oder verzweigten C₂-C₁₀-Glycolen oder Polyglycolen; geradkettigen oder verzweigten C₂-C₄-Monoalkyletherglycolen, wobei der Alkylrest ein C₁-C₄-Alkylrest ist; Dimethoxymethan; Polyalkylenglycolen, ausgewählt aus Polyoxyethylenglycolen mit einem zahlenmittleren Molekulargewicht niedriger als 1000; Polyoxypropylenglycolen mit einem Molekulargewicht niedriger als 2000; Polyoxyethylenpolyoxypropylenglycolen mit einem Molekulargewicht niedriger als 3000.

9. Zusammensetzungen gemäß den Ansprüchen 1 - 8, wobei die X- und/oder O-Phasen pharmazeutische Wirkstoffe zur topischen Anwendung enthalten.

10. Zusammensetzungen gemäß den Ansprüchen 1 - 9, wobei die Gesamtmenge der Phasen X + O, ausgedrückt in Gewichtsprozent der gesamten Zusammensetzung, im Bereich von 20% bis 70% liegt.

11. Zusammensetzungen gemäß den Ansprüchen 1 - 10, wobei in R_{f} des Bestandteils A1) die Wiederholungseinheit -(C₃F₆O)- ausgewählt ist aus -(CF(CF₃)CF₂O- und/oder -(CF₂-CF(CF₃)O)-.

12. Zusammensetzungen gemäß den Ansprüchen 1-11, wobei die (Per)fluorpolyetherkette R_{f} ausgewählt ist aus den folgenden Strukturen:
1)
-(CFO)ₐ-(CF₂CF₂O)_{b}-
wobei b/a zwischen 0,3 bis 10 liegt, die Extremwerte eingeschlossen, wobei a eine ganze Zahl ist, die von 0 verschieden ist;
2)
-(CF₂-(CF₂)_{z'}-CF₂O)_{b'}-
wobei z' eine ganze Zahl gleich 1 oder 2 ist;
3)
-(C₃F₆O)ᵣ-(C₂F₄O)_{b}-(CFLₒO)ₜ-
wobei r/b = 0,5 bis 2,0 ist, (r + b)/t = 10 bis 30 ist, b und t ganze Zahlen sind, die von 0 verschieden sind;
4)
-(OC₃F₆)ᵣ-(CFL₀O)ₜ-OCF₂-R'_{f}-CF₂O-(C₃F₆O)ᵣ-(CFL₀O)ₜ-;
5)
-(CF₂CF₂CH₂O)_{q'}-R'_{f}-O-(CH₂CF₂CF₂O)_{q'}-,
wobei:
R'_{f} ein Fluoralkylenrest mit 1 bis 4 Kohlenstoffatomen ist;
L₀ ausgewählt ist aus F, CF₃;
6)
-(C₃F₆O)ᵣ-OCF₂-R'_{f}-CF₂O-(C₃F₆O)ᵣ-
wobei a, b, b', q', r, t ganze Zahlen sind, deren Summe so ist, dass die (Per)fluorpolyetherkette R_{f} Werte des zahlenmittleren Molekulargewichts Mn zwischen 500 und 5000 aufweist.

13. Zusammensetzungen gemäß Anspruch 12, wobei die (Per)fluorpolyetherkette R_{f} ausgewählt ist aus den folgenden Stukturen:
-(CF₂O)ₐ-(CF₂CF₂O)_{b}-;
-(C₃F₆O)ᵣ-(C₂F₄O)_{b}-(CFL₀O)ₜ-
wobei L₀ und die Indices a, b, r, t den vorstehenden Wert aufweisen.

14. Zusammensetzungen gemäß den Ansprüchen 1-13, wobei der Bestandteil B1) ein Copolymer ist, welches Monomere vom Acryltyp umfasst, erhältlich durch Copolymerisation eines monoolefinischen Carbonsäuremonomers mit einem Ester einer Acrylsäure mit einem C₈-C₃₀-Alkohol.

15. Zusammensetzungen gemäß Anspruch 14, wobei das Carbonsäuremonomer vom Acryltyp die allgemeine Formel
CH₂=CR_{A}-COOH
aufweist, wobei R_{A} ausgewählt ist aus H, Halogen, OH, Lacton, Lactam, CN, einwertigen C₁-C₂₀-Resten, wobei die Reste ausgewählt sind aus Aryl, Arylalkyl oder Alkylaryl, cycloaliphatischen Resten.

16. Zusammensetzungen gemäß den Ansprüchen 14 - 15, wobei die Acrylester die Formel
CH₂=CR₁-COOR₂
aufweisen, wobei R₁ die Bedeutung H, CH₃, C₂H₅ hat; R₂ ein Alkylrest mit 8 bis 30 Kohlenstoffatomen ist oder ein Oxyalkylen- oder Carbonyloxyalkylenrest ist.

17. Zusammensetzungen gemäß den Ansprüchen 14 - 16, wobei die Copolymere auf Acrylsäure und Acrylestern mit einer C₁₀-C₃₀-Alkylkette basieren.

18. Verfahren zum Erhalt der X/O/A-Emulsionen gemäß den Ansprüchen 1-17, umfassend die folgenden Schritte:
- Erwärmen der Phase X, wobei die Additive und die Wirkstoffe, welche in der hydrophilen Flüssigkeit löslich sind, vorher gegebenenfalls bei einer Temperatur von 60°C bis 100°C gelöst wurden;
- Herstellung der Phase O durch Solubilisierung bei einer Temperatur zwischen 50°C und 60°C des bifunktionellen Alkylamid(per)fluorpolyetherderivats der Formel (I), Bestandteil A1) und gegebenenfalls möglicher Additive und/oder Wirkstoffe, welche in dieser Phase löslich sind, in den Estern monofunktioneller C₄-C₁₂-Alkohole, C₂-C₆-Glycole oder C₃-C₆-Polyole, mit C₈-C₃₀-Fettsäuren, gegebenenfalls als Mischung mit anderen Ölen;
- Erwärmen der Phase O bei einer Temperatur im Bereich von 70°C bis 90°C;
- Hinzufügen der Phase X zur Phase O unter Rühren zwischen 1000 und 10000 U/min über 10-20 Minuten und Erhalt der primären Emulsion X/O;
- Abkühlen der primären Emulsion X/O, bis die Emulsionstemperatur zwischen 20°C und 60°C liegt;
- Dispersion des Copolymerbestandteils B1) in die äußere Wasserphase A mit optionalen Exzipienten und anschließendes Hinzufügen der X/O-Emulsion zur Phase A unter Rühren bei mindestens 1000 U/min, Erhalten einer Dispersion,
wobei der Gewichtsprozentsatz der primären Emulsion im Bereich von 10% bis 90% liegt;
- Hinzufügen einer wässrigen Base zur Endzusammensetzung, um einen pH-Wert zwischen 5,0 und 7,5 zu erhalten.

19. Verwendung der Zusammensetzungen gemäß den Ansprüchen 1 - 17, um kosmetische Zusammensetzungen mit wasserabweisender Wirkung herzustellen.

20. Verwendung der Zusammensetzungen gemäß den Ansprüchen 1 - 17 als Grundlagen, um Arzneimittel zur topischen Anwendung herzustellen.

21. Zusammensetzungen gemäß Anspruch 1, wobei A1) 0,1 bis 5 Gew.-% beträgt, R und R', gleich oder verschieden, eine geradkettige oder verzweigte C₁₄-C₁₈-Alkylkette sind.

22. Zusammensetzungen gemäß Anspruch 1, wobei das zahlenmittlere Molekulargewicht von R_{f} von A1) 1000 bis 2500 beträgt.

23. Zusammensetzungen gemäß Anspruch 1, wobei der pH-Wert der Zusammensetzung nicht höher als 7,5 ist.

24. Zusammensetzungen gemäß Anspruch 21, wobei A1) 0,5 bis 3 Gew.-% beträgt.

25. Zusammensetzungen gemäß Anspruch 1 - 8, wobei die X- und/oder O-Phasen Wirkstoffe enthalten, ausgewählt aus Vitamin C und Vitamin E; Ceramiden; essentiellen Ölen.

26. Zusammensetzungen gemäß Anspruch 13, wobei die (Per)fluorpolyetherkette R_{f} die Bedeutung -(CF₂O)ₐ-(CF₂CF₂O)_{b}- hat, wobei a und b die vorstehend angegebene Bedeutung aufweisen.

27. Zusammensetzungen gemäß Anspruch 14, wobei das monoolefinische Carbonsäuremonomer Acrylsäure ist.

28. Zusammensetzungen gemäß Anspruch 15, wobei R_{A} ein einwertiger C₁-C₆-Rest ist.

29. Verfahren gemäß Anspruch 18, wobei das Erwärmen der Phase O bei einer Temperatur im Bereich von 80°C - 90°C durchgeführt wird.

30. Verfahren gemäß Anspruch 18, wobei in der Dispersion, die durch Hinzufügen der X/O-Emulsion zur Phase A erhalten wurde, der Gewichtsprozentsatz der primären Emulsion im Bereich von 30% - 60% liegt.

31. Verfahren gemäß Anspruch 18, wobei nach Hinzufügen der wässrigen Base zur Enddispersion der pH-Wert im Bereich von 5,5 - 7 liegt.

32. Zusammensetzungen gemäß den Ansprüchen 1 bis 8, welche die Wirkstoffe nach Anspruch 25 und pharmazeutische Wirkstoffe zur topischen Anwendung enthalten.

## Revendications

1. Compositions sous la forme d'émulsions multiples ayant une structure X/O/A, dans laquelle X ou phase interne est un liquide hydrophile non miscible avec l'huile ; O est une phase huileuse ; A, phase externe ou phase continue, est une phase aqueuse, lesdites compositions comprenant :
A1) de 0,01 % à 10 % en poids d'un dérivé d'alkylamide (per)fluoropolyéther bifonctionnel ayant la formule :
R'-NH-C (O)-CF₂O-R_{f}-CF₂-C (O)-NH-R (I)
dans laquelle
R et R', équivalents ou différents, sont une chaîne alkyle en C₁₃-C₂₀ linéaire ou ramifiée ;
R_{f} représente une chaîne (per)fluoropolyéther comprenant des motifs répétitifs choisis parmi un ou plusieurs des motifs suivants :
a)
-(C₃F₆O)- ;
b)
-(CF₂CF₂O)- ;
c)
-(CFLₒO)-, où Lₒ = -F, -CF₃ ;
d)
-CF₂(CF₂) _{z'} CF₂O-,
où z' est un entier égal à 1 ou 2 ;
e)
-CH₂CF₂CF₂O- ;
le poids moléculaire moyen en nombre de R_{f} étant de 500 à 5000 ;
B1) de 0,1 % à 0,5 % en poids d'un copolymère, comprenant des monomères de type acrylique, gonflables dans l'eau, **caractérisés par** la présence de groupes acides et de groupes hydrophobes, ayant une activité émulsifiante après la neutralisation des groupes acides avec des bases ;
le pH des compositions étant d'au moins 5 ;
dans lesquelles la phase huileuse O représente de 5 % à 40 % et X+O est compris dans la plage allant de 10 % à 90 % en tant que pourcentage en poids de la composition totale ;
dans lesquelles la phase huileuse O comprend des esters d'alcools en C₄-C₁₂ monofonctionnels, ou des glycols en C₂-C₆ ou des polyols en C₃-C₆, avec des acides gras en C₈-C₃₀, linéaires ou ramifiés, hydrogénés et non hydrogénés, ou leurs mélanges ;
la somme de tous les composants, comprenant les phases X, O et A et les substances dissoutes et/ou dispersées dans celles-ci étant 100 %.

2. Compositions selon la revendication 1, dans lesquelles la phase huileuse représente, en pourcentage en poids, de 10 % à 30 %.

3. Compositions selon les revendications 1 et 2, dans lesquelles la phase huileuse O comprend des huiles minérales ; des huiles hydrocarbonées linéaires ou ramifiées contenant de préférence de 14 à 36 atomes de carbone ; des polymères fluorés liquides choisis parmi les perfluoropolyéthers, les dihydrofluoroéthers et les perfluoroalcanes, la quantité desdites huiles étant inférieure ou égale à 50 % en poids, par rapport au poids total de la phase huileuse.

4. Compositions selon les revendications 1 à 3, dans lesquelles la phase A contient éventuellement des additifs solubles ou dispersibles dans l'eau, de préférence choisis parmi les émulsifiants cationiques, anioniques, non-ioniques ; les modificateurs de rhéologie hydrophiles, les anti-oxydants ; les séquestrants ; les parfums ; les conservateurs ; les colorants ; les poudres minérales ; les pigments ; les polymères solides micronisés ; les filtres solaires ; les agents d'hydratation.

5. Compositions selon les revendications 1 à 4, dans lesquelles la phase X est de l'eau ou une solution aqueuse ou un liquide hydrophile autre que de l'eau.

6. Compositions selon la revendication 5, dans lesquelles la phase X est une solution aqueuse et contient des additifs solubles ou dispersibles dans l'eau choisis parmi les émulsifiants cationiques, anioniques, non-ioniques ; les modificateurs de rhéologie hydrophiles, les anti-oxydants ; les séquestrants ; les parfums ; les conservateurs ; les colorants ; les poudres minérales ; les pigments ; les polymères solides micronisés ; les filtres solaires ; les agents d'hydratation.

7. Compositions selon les revendications 5 et 6, dans lesquelles la phase X contient les additifs suivants : les polyphénols, les hydroxyacides en C₂-C₆ ; les hydroxyacides aromatiques de préférence l'acide salicylique ; l'acide pyroolidone-carboxylique ; les hydrolysats protéiques ; l'acide hyaluronique ; les acides aminés.

8. Compositions selon la revendication 5, dans lesquelles la phase X est un liquide hydrophile autre que de l'eau choisi parmi les glycols ou les polyglycols en C₂-C₁₀ linéaires ou ramifiés ; les monoalkyléther glycols en C₂-C₄ linéaires ou ramifiés dans lesquels le groupe alkyle est un groupe en C₁-C₄; le diméthoxyméthane ; les polyalkylène glycols choisis parmi les polyoxyéthylène glycols ayant un poids moléculaire moyen en nombre inférieur à 1000 ; les polyoxypropylène glycols ayant un poids moléculaire inférieur à 2000 ; les polyoxyéthylènepolyoxypropylène glycols ayant un poids moléculaire inférieur à 3000.

9. Compositions selon les revendications 1 à 8, dans lesquelles les phases X et/ou O contiennent les principes actifs pharmaceutiques pour une utilisation locale.

10. Compositions selon les revendications 1 à 9, dans lesquelles la quantité totale des phases X + O, exprimée en pourcentage en poids du total de la composition, est comprise dans la plage allant de 20 % à 70 %.

11. Compositions selon les revendications 1 à 10, dans lesquelles dans R_{f} du composant A1), le motif répétitif (-C₃F₆O)- est choisi parmi -(CF(CF₃)CF₂O)- et/ou -(CF₂-CF(CF₃)O)-

12. Compositions selon les revendications 1 à 11, dans lesquelles la chaîne (per)fluoropolyéther R_{f} est choisie parmi les structures suivantes :
1)
-(CF₂O)ₐ-(CF₂CF₂O)_{b}-
b/a étant compris entre 0,3 et 10, les extrêmes inclus, a étant un entier différent de 0 ;
2)
-(CF₂-(CF₂)_{z'}-CF₂O)_{b'}-
où z' est un entier égal à 1 ou 2 ;
3)
-(C₃F₆O)ᵣ-(C₂F₄O)_{b}-(CFLₒO)ₜ-
r/b = 0,5-2,0, (r+b)/t = 10-30, b et t étant des entiers différents de 0;
4)
-(OC₃F₆)ᵣ-(CFLₒO)ₜ-OCF₂-R'_{f}-CF₂O-(C₃F₆O)ᵣ-(CFLₒO)ₜ;-
5)
-(CF₂CF₂CH₂O)_{q}-R'_{f}-O-(CH₂CF₂CF₂O)_{q}-
où R'_{f} est un groupe fluoroalkylène de 1 à 4 atomes de carbone;
Lₒ est choisi parmi F, CF₃;
6)
-(C₃F₆O)ᵣ-OCF₂- R'_{f}-CF₂O-(C₃F₆O)ᵣ-;
a, b, b', q', r, t étant des entiers, dont la somme est telle que la chaîne (per)fluoropolyéther R_{f} a un poids moléculaire moyen en nombre Mn compris entre 500 et 5000.

13. Compositions selon la revendication 12, dans lesquelles la chaîne (per)fluoropolyéther R_{f} est choisie parmi les structures :
-(CF₂O)ₐ-(CF₂CF₂O)_{b}-;
-(C₃F₆O)ᵣ(C₂F₄O)_{b}-(CFLₒO)ₜ-;
où Lₒ et les indices a, b, r, t ont la valeur ci-dessus.

14. Compositions selon les revendications 1 à 13, dans lesquelles le composant B1) est un copolymère comprenant des monomères de type acrylique pouvant être obtenus par copolymérisation d'un monomère monooléfinique carboxylique avec un ester d'un acide acrylique avec un alcool en C₈-C₃₀.

15. Compositions selon la revendication 14, dans lesquelles le monomère carboxylique de type acrylique a la formule générale :
CH₂=CR_{A}-COOH
dans laquelle R_{A} est choisi parmi H, un atome d'halogène, OH, une lactone, un lactame, CN, des radicaux monovalents en C₁-C₂₀, lesdits radicaux étant choisis parmi les radicaux aryle, arylalkyle ou alkylaryle, et des radicaux cycloaliphatiques.

16. Compositions selon les revendications 14 et 15, dans lesquelles les esters acryliques ont la formule:
CH₂=CR₁-COOR₂
dans laquelle R₁ est H, CH₃, C₂H₅ ; R₂ est un groupe alkyle de 8 à 30 atomes de carbone, ou un groupe oxyalkylène ou carbonyloxyalkylène.

17. Compositions selon les revendications 14 à 16, dans lesquelles les copolymères sont à base d'acide acrylique et d'esters acryliques ayant une chaîne alkyle en C₁₀-C₃₀.

18. Procédé pour obtenir les émulsions X/O/A selon les revendications 1 à 17 comprenant les étapes suivantes :
- chauffer la phase X, dans laquelle les additifs et les principes actifs solubles dans ledit liquide hydrophile ont été préalablement éventuellement dissous à une température de 60°C à 100°C;
- préparer la phase O par solubilisation à une température entre 50 °C et 60 °C des dérivés d'alkylamide (per)fluoropolyéther bifonctionnels du composant A1) de formule I), et éventuellement les additifs et/ou principes actifs possibles solubles dans ladite phase, dans les esters d'alcools en C₄-C₁₂ monofonctionnels, les glycols en C₂-C₆ ou les polyols en C₃-C₆, avec des acides gras en C₈-C₃₀, éventuellement en mélange avec d'autres huiles ;
- chauffer la phase O à une température dans la plage de 70 °C à 90°C;
- ajouter la phase X à la phase O sous agitation entre 1000 et 10000 tours/minute pendant 10 à 20 minutes et obtenir la première émulsion X/O;
- refroidir la première émulsion X/O jusqu'à ce que la température de l'émulsion soit comprise entre 20 °C et 60 °C ;
- disperser le composant copolymère B1) dans la phase aqueuse externe A avec des excipients facultatifs et ajouter ensuite l'émulsion X/O à la phase A, sous agitation d'au moins 1000 tours/minute, donnant une dispersion dans laquelle le pourcentage en poids de la première émulsion est compris dans la plage allant de 10 % à 90 % ;
- ajouter à la composition finale une base aqueuse pour obtenir une valeur de pH entre 5,0 et 7,5.

19. Utilisation des compositions selon les revendications 1 à 17 pour préparer des compositions cosmétiques ayant une activité hydrophobe.

20. Utilisation des compositions selon les revendications 1 à 17 en tant que bases pour préparer des compositions pharmaceutiques pour une utilisation locale.

21. Compositions selon la revendication 1, dans lesquelles A1) est de 0,1 % à 5 % en poids, R et R', identiques ou différents, sont une chaîne alkyle en C₁₄-C₁₈ linéaire ou ramifiée.

22. Compositions selon la revendication 1, dans lesquelles le poids moléculaire moyen en nombre de R_{f} de A1) est de 1000 à 2500.

23. Compositions selon la revendication 1, dans lesquelles le pH de la composition est inférieur ou égal à 7,5.

24. Compositions selon la revendication 21, dans lesquelles A1) est de 0,5 % à 3 % en poids.

25. Compositions selon les revendications 1 à 8, dans lesquelles les phases X et/ou O contiennent des principes actifs choisis dans le groupe de la vitamine C et de la vitamine E ; des céramides ; des huiles essentielles.

26. Compositions selon la revendication 13, dans lesquelles la chaîne (per)fluoropolyéther R_{f} est -(CF₂O)ₐ-(CF₂CF₂O)_{b}-, où a et b ont la signification indiquée ci-dessus.

27. Compositions selon la revendication 14, dans lesquelles le monomère carboxylique monooléfinique est l'acide acrylique.

28. Compositions selon la revendication 15, dans lesquelles R_{A} est un radical monovalent en C₁-C₆.

29. Procédé selon la revendication 18, dans lequel le chauffage de la phase O est réalisé à une température comprise dans la plage allant de 80°C à 90 °C.

30. Procédé selon la revendication 18, dans lequel dans la dispersion obtenue en ajoutant l'émulsion X/O à la phase A, le pourcentage en poids de la première émulsion est compris dans la plage allant de 30 % à 60 %.

31. Procédé selon la revendication 18, dans lequel après l'ajout de la base aqueuse à la dispersion finale, le pH est compris dans la plage allant de 5,5 à 7.

32. Compositions selon les revendications 1 à 8, contenant les principes actifs selon la revendication 25 et des principes actifs pharmaceutiques pour une utilisation locale.
